(19) **Europäisches Patentamt**

**European Patent Office**

**Office européen des brevets**

(11) **EP 0 613 926 B1**

(12) **EUROPÄISCHE PATENTSCHRIFT**

(45) Veröffentlichungstag und Bekanntmachung des Hinweises auf die Patenterteilung:
**07.06.2000 Patentblatt 2000/23**

(51) Int. Cl.[7]: **C08L 71/00**, A61K 6/10

(21) Anmeldenummer: **94103248.4**

(22) Anmeldetag: **04.03.1994**

(54) **Hydrophilierte Polyether**

Hydrophilized polyethers

Polyéthers hydrophilisés

(84) Benannte Vertragsstaaten:
**AT BE CH DE FR GB IT LI**

(30) Priorität: **05.03.1993 DE 4306997**

(43) Veröffentlichungstag der Anmeldung:
**07.09.1994 Patentblatt 1994/36**

(73) Patentinhaber: **ESPE Dental AG
82229 Seefeld (DE)**

(72) Erfinder:
• **Guggenberger, Rainer, Dr.
D-82211 Herrsching (DE)**
• **Wanek, Erich, Dr.
D-86916 Kaufering (DE)**
• **Gasser, Oswald, Dr.
D-82229 Seefeld (DE)**

(74) Vertreter:
**Abitz, Walter, Dr.-Ing. et al
Patentanwälte Abitz & Partner
Postfach 86 01 09
81628 München (DE)**

(56) Entgegenhaltungen:
**EP-A- 0 421 371        EP-A- 0 480 238
DE-A- 3 246 654        DE-A- 4 019 249
DE-A- 4 119 484        US-A- 4 657 959**

• **DATABASE WPI Week 9248, Derwent
Publications Ltd., London, GB; AN 92-394631 &
JP-A-4 293 955 (TOKUYAMA SODA KK) 19.
Oktober 1992**

**Beschreibung**

Die vorliegende Erfindung betrifft hydrophilierte gummielastische Abform- bzw. Dubliermassen auf Basis von vulkanisierbaren Polyethermaterialien, die insbesondere im Dentalbereich, aber auch in der Orthopädie, Anwendung finden, ein Verfahren zu ihrer Herstellung und ein geeignetes Mittel.

Die vorliegende Erfindung betrifft insbesondere vulkanisierbare Polyetherpasten mit Aziridinogruppen zur Herstellung genauer Abformungen bezahnter, teilbezahnter und unbezahnter Kiefer sowie von Gipsmodellen.

Derartige Systeme sind an sich bekannt.

Für die verschiedenen Abformmethoden werden die Massen in verschiedenen Viskositätsklassen angeboten, so beispielsweise die knetbaren und hochviskosen Massen für die Löffel- und die mittel- und niedrigviskosen Massen vorzugsweise für die Spritzenapplikation.

Aus der DE-B-17 45 810 sind Abdruckmassen von Polyethermaterialien mit Aziridinoendgruppen bekannt.

Aus der DE-A1-37 41 575 und der DE-A1-38 38 587 sind Abdruckmassen auf Basis von Polyethermaterialien mit Alkenylgruppen sowie Polyorganosiloxanresten, enthaltend H-Si-Gruppen, bekannt, die unter der Einwirkung von Platinkatalysatoren polymerisieren.

Aus der EP-A2-0 173 085 sind Abdruckmassen von Polyethermaterialien mit Acrylat- und Methacrylatgruppen bekannt, die nach Bestrahlung mit Licht geeigneter Wellenlänge - initiiert durch den Zerfall eines Photoinitiators - polymerisieren.

Diese Materialien haben ein gutes Anfließvermögen an hydrophilen, oralen Flächen und damit bei diesen Anwendungen eine höhere Abdruckschärfe als andere bekannte Abdruckmassen, z.B. auf Basis von konventionellen hydrophoben Silikonen.

Nachteil dieser bekannten Polyethermaterialien ist jedoch, daß ihre Wasseraufnahme im Vergleich zu Silikonabdruckmassen höher ist. Durch die zur Unterbrechung der Infektionskette inzwischen unerläßliche, häufig auch mehrmalige, Desinfektion von Abdruckmassen mittels wäßriger Desinfektionsbäder werden heute aber erhöhte Anforderungen an eine möglichst geringe Wasseraufnahme, geringe Quellung und damit niedrige Dimensionsänderung gestellt.

Ein weiterer Nachteil der bekannten Polyethermaterialien ist ihr höherer Randwinkel verglichen mit anderen bekannten Abdruckmassen, z.B. solchen auf der Basis von Hydrokolloiden. Letztere weisen allerdings eine schlechtere Formstabilität auf als Polyethermaterialien. Infolge ihrer hohen Anfälligkeit gegenüber Quellung oder Schrumpfung sind die Hydrokolloide nicht lagerfähig; die Abdrücke müssen sofort ausgegossen werden.

Ein höherer Randwinkel kann beim Ausgießen des Abdrucks mit Gipssuspension zu vermindertem Anfließen und zum Verbleib von Lufteinschlüssen führen. Derartige Gipsmodelle sind unbrauchbar. Der Randwinkel ist der Winkel, den der Rand eines Wassertropfens zur Substratoberfläche bildet (Walter Noll, Chemistry and Technology of Silicons, Academic Press, 1968, insbesondere die Seiten 447-452).

Die weiterhin bekannten Silikonabdruckmassen haben zwar den Vorteil einer relativ geringen Wasseraufnahme, ihr Anfließvermögen und ihr Randwinkel sind jedoch nicht zufriedenstellend. Man hat deshalb versucht, diesen Silikonmassen durch Einverleibung eines Hydrophilierungsmittels einen hydrophileren Charakter zu verleihen und ihren Randwinkel zu erniedrigen. Solche Massen sind beispielsweise in der EP-A1-0 480 238 beschrieben. Ein wesentlicher Nachteil der hydrophilierten Silikonmassen besteht jedoch darin, daß der durch Zusatz der Hydrophilierungsmittel erreichte niedrigere Randwinkel durch bereits einmalige Desinfektion im wäßrigen Desinfektionsbad weitgehendst verlorengeht.

Besonders nachteilig ist die erhöhte Wasseraufnahme hydrophiler Silikone, die ein Vielfaches der nicht hydrophilierten Silikone beträgt. Gribi, Quintessenz, Zahntech. 18, 1261-1274 (1992) beschreibt in Abb. 7, Seite 1271 Probekörper gleicher Größe, die 8 Stunden fließendem bzw. stehendem Wasser ausgesetzt wurden. Nach oberflächlicher Trocknung wurden die Proben gewogen. Als Ergebnis wurde gefunden, daß das hydrophobe Silikon (Coltene President Jet Lightbody) 0,12 % Wasseraufnahme zeigt. Das hydrophilierte Silikon (Coltene President Plus Jet Lightbody) nimmt 0,40 % Wasser auf; dies entspricht einer Steigerung um das 3,3-fache des hydrophoben Silikons. Ein hydrophiliertes Silikon mit extrem tiefem Randwinkel nimmt 1,13 % Wasser auf, dies entspricht einer Steigerung um das 9,4-fache des hydrophoben Silikons. Ein besonderer Nachteil der erhöhten Wasseraufnahme liegt in der dadurch bei Silikonen ausgelösten Wasserstoffentwicklung aus der Vernetzerkomponente, die zu Blasen im Modell führt. Nachteilig ist schließlich auch die verzögerte Abbindung solchermaßen hydrophilierter Silikone, so daß die Entfernung des Abdrucks vor der Beendigung der Härtungsreaktion eventuell zu einer Verformung des Abdrucks führen kann, und daß ihre Abbindung durch Latexhandschuhe, die aus hygienischen Gründen in der zahnärztlichen Behandlung unverzichtbar sind, inhibiert wird.

Aufgabe der Erfindung ist es, Abdruckmassen zur Verfügung zu stellen, die die oben geschilderten Nachteile nicht aufweisen, und die insbesondere auch bei mehrmaliger Desinfektion eine zufriedenstellende Dimensionsstabilität, d.h. eine zufriedenstellende geringe Wasseraufnahme, und einen möglichst niedrigen und unverändert bleibenden Randwinkel aufweisen.

Gelöst wird diese Aufgabe durch gummielastische Massen auf der Basis vulkanisierbarer Polyether mit Aziridinogruppen, welche dadurch gekennzeichnet sind, daß sie in fertig vulkanisierter und auspolymerisierter Form 0,1 bis 15 Gew.-%, bezogen auf das Gesamtgewicht der Masse, wenigstens eines eine hydrophile Natur verleihenden Mittels aus der Gruppe bestehend aus hydrophilen Silikonölen, fluorierten Kohlenwasserstoffen, Blockcopolymeren von Ethylenoxid/Propylenoxid, Fettalkoholderivaten, Alkylphenolderivaten, Fettaminen, Aminoxiden, Fettsäureglykol- und -glycerinderivaten, Fettsäuren und Fettsäuremonoestern enthalten.

Überraschenderweise wurde gefünden, daß durch den Zusatz wenigstens einer der oben genannten Stoffe die Hydrophilie der Vulkanisate auf Polyetherbasis bedeutend verbessert werden kann und diese damit einen niedrigen Randwinkel aufweisen. Dennoch besitzen die erfindungsgemäßen Massen keine oder eine nur unwesentlich erhöhte, für die Anwendung im Dentalbereich unschädliche Wasseraufnahme, Quellung und Dimensionsänderung und zeichnen sich deshalb durch erhöhte Wiedergabegenauigkeit aus. Dies ist im Hinblick auf die Erfahrungen mit den hydrophilierten Silikonabdruckmassen besonders überraschend. Völlig unerwartet ist auch, daß der durch den erfindungsgemäßen Zusatz erreichte niedrige Randwinkel auch nach mehrmaliger Desinfektion in einem wäßrigen Desinfektionsbad erhalten bleibt. Von überraschendem. Vorteil ist darüber hinaus, daß durch den erfindungsgemäßen Zusatz auch die Hydrophilie der angemischten, noch nicht vulkanisierten Massen, bedeutend verbessert werden kann und diese einen niedrigen Randwinkel aufweisen.

Von besonderem Vorteil ist schließlich, daß sich der Zusatz geeigneter erfindungsgemäßer Hydrophilierungsmittel ohne schädliche Auswirkung auf die Verarbeitungs- und Abbindezeit, den Abbindeübergang, die sonstigen physikalischen Werte und die Lagerstabilität erweist.

Die erfindungsgemäße gummielastische Masse enthält

- als Polymerisat bzw. Copolymerisat mindestens einen vulkanisierbaren Polyether mit Aziridinogruppen

- für Polyether übliche Polymerisationskatalysatoren (siehe beispielsweise US-A-4 167 618), Aktivatoren, Beschleuniger und Verzögerer, Stabilisatoren, gegebenenfalls gelöst oder suspendiert in dafür üblichen Lösungsmitteln oder Weichmachern,

- wenigstens ein Hydrophilierungsmittel der oben angegebenen Art,

- gegebenenfalls weitere übliche Weichmacher, Lösungsmittel, Suspensionshilfsmittel, pyrogene oder gefällte Kieselsäuren, weitere übliche Füllstoffe, Desinfektionsmittel und weitere übliche Zusatzstoffe.

Unter dem Begriff vulkanisierbare Polyether mit Aziridinogruppen werden Materialien mit Polyethermittelstücken und reaktiven Aziridinogruppen verstanden, die bevorzugt endständig und/oder aber auch im Polyethermittelstück vorhanden sein können.

Besonders bewährt haben sich zur Herstellung des Ausgangsmaterials Polyglykolether. In Frage kommen z.B. Polymerisate und Mischpolymerisate cyclischer Ether, insbesondere mit 3 bis 5 Ringgliedern, wie Ethylenoxid, Propylenoxid, Tetrahydrofuran, Oxethan (Trimethylenoxid), sowie Substitutionsprodukte. Neben unverzweigten Produkten kommen auch schwachverzweigte Produkte oder verzweigte Produkte in Frage, wie Oxyethylierungsprodukte von drei- oder mehrwertigen Alkoholen.

Das Polyethermittelstück kann aber auch einen aromatisch ungesättigten heterocyclischen oder cycloaliphatischen Ring enthalten, oder auch ein siloxan-substituierter Polyether sein.

Sämtliche genannten Typen von Polyglykolethern können in Folgereaktionen mit den reaktiven Aziridinogruppen ausgestattet werden, indem zunächst Substituenten eingeführt werden, die dann ihrerseits zur Reaktion mit geeigneten reaktive Gruppen verleihenden Derivaten fähig sind.

Die Aziridinogruppen sind funktionelle Gruppen, die nach geeigneter Initiierung durch Starter zur Polymerisation der Massen führen.

Beispiele fur geeignete Polyether mit Aziridinogruppen sind in der DE-B-17 45 810 beschrieben. Der Inhalt der genannten Druckschrift soll hier ausdrücklich mitumfaßt sein.

Bevorzugte Starter dafür sind in der DE-A1-25 15 593 und geeignete Verzögerer in der EP-A1-0 110 429 beschrieben.

Um eine vorzeitige Härtung der Abformmassen zu verhindern, müssen die Polyethermaterialien einerseits und die Katalysatoren andererseits bis zum Gebrauch voneinander getrennt gehalten werden (eine Ausnahme davon bilden nur die lichthärtenden Polyethermassen). Vorzugsweise liegen beide Komponenten in Form von Pasten vor.

Die vulkanisierbaren Polyethermaterialien der einen Paste können zur Verbesserung des Anmischverhaltens mit dem im Lösungsmittel oder Weichmacher gelösten oder mittels Suspendierhilfsmittel in Suspension oder mittels Emulgatoren in Emulsion gehaltenen Polymerisationskatalysator der anderen Paste, gleichartige oder chemisch ähnliche Weichmacher oder Lösungsmittel enthalten.

Die Verwendung von Lösungen des Vernetzungsmittels in geeigneten Weichmachern, Lösungsmitteln oder Weichmacher-Gemischen ist zweckmäßig; auf diese Weise werden nicht nur extreme Mischungsverhältnisse vermieden, sondern auch feste Starter gelöst.

Zur Vermeidung des Nachteils leicht beweglicher Vernetzungs- bzw. Härtungsmittel können diese in eine entsprechende viskose Form gebracht werden, z.B. durch Zugabe von Kunststoff, wie Polyvinylacetat, oder durch Einarbeitung von Füllmitteln mit großer Oberfläche, wie hochdisperse Kieselsäure.

Übliche Weichmacher sind häufig mit den Polyethermaterialien gut verträglich. Ihre Verwendung ist nicht nur aus wirtschaftlichen Gründen, sondern auch zur Verbesserung der Eigenschaften, insbesondere zur Vermeidung oder Verringerung der Kristallisation ratsam. Geeignet sind beispielsweise Phthalsäureester, Glykolderivate, sowie polymere Weicher, Sorbitanester, etc. Übliche und geeignete Weichmacher sind beispielsweise in Polyethers, Part I, herausgegeben von Norman G. Gaylord, Interscience Publishers (1963) beschrieben.

Die Eigenschaften der Endprodukte sind durch Wahl eines geeigneten Ausgangsmaterials weitgehend variabel, so daß die mechanischen Werte der Endprodukte nahezu nach Maß eingestellt werden können. Der Zusatz größerer Mengen Weichmacher und/oder weiterer üblicher Zusatzstoffe allerdings kann aber die Wasseraufnahme, Quellung und Dimensionsänderung in einem Ausmaß beeinflussen, daß der Abdruck unbrauchbar ist.

Die erfindungsgemäßen Massen enthalten in fertig vulkanisierter und auspolymerisierter Form 0,1 bis 15, vorzugsweise 0,3 bis 10 und insbesondere 0,5 bis 5 Gew.-%, bezogen auf das Gesamtgewicht der Massen, wenigstens eines eine hydrophile Natur verleihenden Mittels, ausgewählt aus der Gruppe bestehend aus fluorierten Kohlenwasserstoffen, Blockcopolymeren von Ethylenoxid/Propylenoxid, Fettalkoholderivaten, Alkylphenolderivaten, Fettaminen, Aminoxiden, Fettsäureglykol- und -glycerinderivaten, Fettsäuren und Fettsäuremonoestern.

Diese Mittel werden entweder der Polyetherbasispaste oder der Katalysatorpaste alleine oder anteilig der Polyetherbasispaste und der Katalysatorpaste in den angegebenen Mengen zugesetzt. Unter 0,1 Gew.-% ist die Hydrophilie des Polyethermaterials nicht verbesserbar, über 15 Gew.-% wird die Wasseraufnahme zu hoch.

Das Hydrophilierungsmittel ist in so ausreichender Menge vorhanden, daß das Vulkanisat einen 10 Sekunden Randwinkel kleiner 55° aufweist. Vorzugsweise weisen die erfindungsgemäßen Massen einen 10-Sekunden-Randwinkel kleiner 45°, besonders bevorzugt kleiner 35° auf.

Unter einem eine hydrophile Natur vermittelnden Mittel versteht man in diesem Zusammenhang ein Hydrophilierungsmittel, das den Randwinkel eines Tropfens Wasser oder wäßriger Zusammensetzung (z.B. Gipssuspension, Speichellösung, Desinfektionslösung, etc.) gegenüber der Polyetherzusammensetzung erniedrigt und damit eine bessere Benetzbarkeit der Polyetherzusammensetzung durch die Flüssigkeit hervorruft. Zur Definition des Randwinkels wird auf Walter Noll, Chemistry and Technology of Silicons, Academic Press, 1968, insbesondere Seiten 447 bis 452 verwiesen. Wie schon oben erwähnt, ist der Randwinkel, oder auch Kontaktwinkel genannt, der Winkel, den der Rand eines Wassertropfens zur Substratoberfläche bildet. Bei hydrophoben Substraten liegt dieser Winkel teilweise über 100°. Je hydrophiler das Substrat ist, desto flacher wird die Form des Tropfens und desto kleiner der Randwinkel.

Die Messung des Randwinkels erfolgt mittels Meßmikroskop am liegenden Tropfen 10 Sekunden nach Aufbringen eines Tropfens einer gesättigten Lösung von Calciumsulfatdihydrat bei 23°C 30 Minuten nach Anmischbeginn. Der 10-Sekunden-Wert für den Kontaktwinkel ist deshalb methodisch vorteilhaft, da der Einfluß von Verdunstungsphänomenen dabei unterbleibt. Verdunstungen würden eine irreführende Veränderung des Randwinkels, d.h. eine Verkleinerung, bewirken. Das Meßverfahren erfolgt im wesentlichen nach der in der Arbeit von Bader und Setz, Benetzbarkeit und Wiedergabegenauigkeit von Abformmassen (Dtsch. Zahnärztl. Z. 46, 346-348 (1991) beschriebenen Methode.

Geeignete Hydrophilierungsmittel aus der Gruppe der hydrophilen Silikonöle enthalten vorzugsweise eine oder mehrere Siloxangruppen als hydrophoben Teil sowie eine oder mehrere Ethergruppierungen als hydrophilen Teil. Diese Ethergruppierungen sind bevorzugt Ethylenoxy- oder Propylenoxy-, aber auch z.B. hydroyalkyl-substituierte Ethylenoxyruppen. Diese Alkylenoxygruppe kann sowohl über ein Kohlenstoffatom oder auch über ein Sauerstoffatom chemisch an die Siloxangruppe gebunden sein. Solche hydrophilen Silikonöle werden in den US-Patenten 3 505 377, 3 980 688, 4 431 789 sowie in der Union Carbide-Information über die Silwet-Produkte beschrieben. Bevorzugte Stoffe sind die darin genannten Typen Silwet L-77, Silwet L-7604, Silwet L-7001, Silwet L-7600 und Silwet L-7602. Weitere Vertreter dieser hydrophilen Silikonöle finden sich in der Produktinformation der Firma Olin Corporation (Silfac-Produkte) und in den US-Patenten 4 160 776, 4 226 794 und 4 337 168.

Geeignete Hydrophilierungsmittel aus der Gruppe der fluorierten Kohlenwasserstoffverbindungen sind beispielsweise diejenigen, die im US-Patent 2 915 544 beschrieben sind. Weitere Vertreter dieser Gruppe sind F-Alkyl-2-ethyl-thiopolyethylenglykol-ethoxylate verschiedener Ethylenoxid(EO) Anzahl.

Erfindungsgemäß geeignete Hydrophilierungsmittel sind weiterhin die Blockcopolymeren des Propylenoxids und Ethylenoxids, wie etwa Derivate des Propylenglykols und des Ethylendiamins, welche von Firma Wyandotte unter den Bezeichnungen Pluronics und Tetronics vertrieben werden. Ethoxylierte Polypropylenoxide unterschiedlicher Kettenlängen sind auch als Synperonic-Typen erhältlich.

Eine weitere Gruppe geeigneter Hydrophilierungsmittel stellen die Fettalkoholderivate, insbesondere die ethoxylierten Fettalkoholderivate dar, wie Fettalkoholpolyglykolether, Laurylalkohol- und Talgfettalkoholethoxylate, Octadecanoloxyethylat, Dodecanol-, Tetradecanol-oxyethylat mit verschiedenen EO-Anteilen. Geeignet sind auch Trimethylnonanolethoxylate.

Weiterhin geeignete Hydrophilierungsmittel kommen aus der Gruppe der Alkylphenolderivate, insbesondere der ethoxylierten Alkylphenole, wie Nonylphenolpolyglykolether, etwa Nonylphenol mit 3 bis 14 EO, Octylphenol- und Polyoxyethylennonylphenolethoxylate.

Aus der Gruppe der Fettamine sind die ethoxylierten Fettamine bevorzugt, so z.B. Fettaminethoxylat.

Weiterhin geeignete Hydrophilierungsmittel sind die Fettsäureglykol- und -glycerinderivate, Fettsäuren und Fettsäuremonoester. Bevorzugt sind dabei die ethoxylierten Fettsäuren und deren Ölsäure-polyglykolester, Kokosfettsäuremonoglyceridpolyglykolether, Talgfettsäuremonoglyceridpolyglykolether. Weitere bevorzugte Vertreter sind Fettsäureester wie Polyethylenglykoldistearat, Glycerinmonostearat, 2-Ethylhexylpalmitat, Butylstearat, Isobutylstearat, Isodecylstearat, Isopropyloleat.

Weitere geeignete Hydrophilierungsmittel sind auch Aminoxide wie Lauryldimethylaminoxid und Stearyldimethylaminoxid.

Besonders bevorzugt werden als Hydrophilierungsmittel Fettalkoholalkoxylate, insbesondere Fettalkoholethoxylate. Die alkoxylierten Fettalkohole und acylierten alkoxylierten Fettalkohole sind gerad- und verzweigtkettige Alkohole von C10 bis C16, welche mit 2 bis 10 Mol Alkylenoxid umgesetzt und gegebenenfalls anschließend mit einer C2 bis C4 Monocarbonsäure verestert wurden. Besonders bevorzugt werden weiter die beschriebenen alkoxylierten Silikonderivate sowie die Blockcopolymeren des Propylenoxids/Ethylenoxids.

Die geeigneten Hydrophilierungsmittel sind nicht auf die in den Beispielen genannten HLB-Werte beschränkt (siehe Definition Seite 15). Weitere spezielle Beispiele der erfindungsgemäß einsetzbaren Hydrophilierungsmittel sind beispielsweise im "Index der Non-Ionic-Surfactants" von Bernard Parant, 1988 sowie in Ullman's Encyklopädie der technischen Chemie, Band 22, 4. Auflage, 1982 beschrieben.

Die erfindungsgemäßen vulkanisierbaren Polyethermaterialien eignen sich insbesondere zum Zwecke der dentalen Abformung und Abdrucknahme. Sie eignen sich aber auch als Dubliermaterialien und Abdruckmaterialien für technische und medizinische Zwecke, z.B. in der Orthopädie zur Abformung der Femur-Kavität vor Hüftgelenksendoprothetik.

Die nachfolgenden Beispiele dienen der Erläuterung der Erfindung. Die Erfindung ist nicht auf die Beispiele beschränkt.

Beispiele

Die Messung des Randwinkels erfolgt mit einem Kontaktwinkelmeßsystem G1/G40 (Krüss). Das Randwinkelmeßgerät G1 ermöglicht die präzise Abbildung von Tropfenprofilen auf Festkörperoberflächen. Das G40-Meßsystem umfaßt einen Videotubus mit Strahlenteiler, so daß die gleichzeitige Beobachtung eines Tropfens durch das Goniometerokular (Tropfengröße) und die Videokamera (digitale Bildauswertung) ermöglicht wird.

Die Messung erfolgt am liegenden Tropfen bei 23°C und 50 % relativer Luftfeuchtigkeit. 30 Minuten nach Anmischbeginn der Massen wird ein stets gleich großer Tropfen einer bei 23°C gesättigten Calciumsulfatdihydratlösung auf das zwischen Glasplatten zu glatter Oberfläche ausgehärtete Elastomer aufgetragen und sofort mit der Messung begonnen. Der 10-Sekunden-Wert wird zur Auswertung herangezogen.

Die Ergebnisse im Vergleich sind in Tabellen 1 bis 3 zusammengefaßt.

Die Bestimmung der Wasseraufnahme erfolgt mittels einer rechteckigen Platte (L = 38, B = 32, H = 3 mm) mit 28,5 cm$^2$ benetzbarer Oberfläche. 60 Minuten nach Anmischbeginn wird das Vulkanisat gewogen (Meßwert a) und in destilliertem Wasser 20 Stunden bei 23°C belassen. Sofort nach Entnahme wird der Prüfkörper oberflächlich getrocknet und erneut gewogen (Meßwert b). Die Wasseraufnahme in Prozent errechnet sich nach der Formel

$$\text{Aufnahme-\%} = [\,(b - a) / a\,] \times 100$$

Das Wasserrückhaltevermögen wurde durch eine erneute Wägung des Prüfkörpers (Meßwert c) nach weiterer 2-stündiger Lagerung des Prüfkörpers an Luft bei 50 % relativer Luftfeuchtigkeit bei 23°C bestimmt. Das Wasserrückhaltevermögen in % errechnet sich nach der Formel

$$\text{Rest-\%} = [\,(c - a) / a\,] \times 100$$

Die Dimensionsänderung wurde in Anlehnung an ADA (American Dental Association) 19 bei 100 % relativer Luftfeuchtigkeit nach 20 Stunden gemessen.

Die Ergebnisse im Vergleich sind in Tabelle 1 aufgeführt.

Die Tauchbad-Desinfektionen werden entsprechend der Gebrauchsinformation des Herstellers in kommerziell erhältlichen Desinfektionslösungen durchgeführt. Für Impresept (ESPE) werden pro Desinfektionsvorgang 10 Minuten Verweilzeit angesetzt, für Banicide (Pascal) ebenso 10 Minuten, für CoeCide XL plus (GC America) hingegen 20 Minuten.

Die Ergebnisse im Vergleich sind Tabelle 3 aufgeführt.

In den Beispielen werden als Hydrophilierungsmittel die folgenden nicht-ionogenen Tenside verwendet:

- PLURONIC Blockcopolymer-Surfactants (BASF Wyandott),
- SILWET Polyalkylenoxid-polymethylsiloxane (Union Carbide Corp.),
- REWOPAL Fettalkoholpolyalkylenoxidmethylether (REWO),
- ANTAROX Nonylphenolethoxylate (Rhone-Poulenc),
- FLUORAD Fluortenside (3M).

Sofern bekannt, wird in den Beispielen auf den jeweiligen HLB-Wert der oberflächenaktiven Stoffe verwiesen. Unter dem HLB-Wert (engl. Hydrophilic Lipophilic Balance) versteht man ein Maß für die Wasser- und Fettfreundlichkeit von nicht-ionogenen Tensiden. Der HLB-Wert läßt sich sowohl experimentell bestimmen als auch durch Rechenverfahren in bestimmten Fällen errechnen. Im allgemeinen liegen die Zahlenwerte zwischen 1 und 20, in seltenen Fällen auch darüber (bis 40). Substanzen mit niedrigem HLB-Wert (etwa unter 10) sind im allgemeinen gute W/O-Emulgatoren, während die mehr wasserfreundlichen Tenside mit höherem HLB-Wert als O/W-Emulgatoren wirken. Somit läßt sich aus der Kenntnis des HLB-Werts auf das Verhalten anderer Tenside schließen.

Beispiel 1 (Vergleichsbeispiel)

Es werden 46,6 g eines Aziridinopolyethers, der gemäß Beispiel 12 der DE-B-17 45 810 erhalten wurde, mit 21,5 g Dibenzyltoluol, 9,64 g hydriertem Palmöl und 22,3 g Kieselgur vermischt. Auf diese Weise werden 100 g einer Paste A erhalten. Weiterhin werden 33,4 g Dibenzyltoluol, 15,6 g 2,5-Dichlorbenzolsulfonsäuremethylester, 15,1 g Polyisobutylen, 10,0 g pyrogene Kieselsäure und 25,9 g Kieselgur zu 100 g einer Paste B verknetet.

Die beiden Pasten werden im Gewichtsverhältnis 4 A : 1 B vollständig miteinander vermischt. Nach einigen Muten erhält man eine gummielastische Masse. 30 Minuten nach Herstellung wird der Randwinkel zu 64° bestimmt. Die Wasseraufnahme beträgt 1,05 %, das Wasserrückhaltevermögen 0,18 %, die Dimensionsänderung +/- 0,00 %.

Beispiel 2

100 g Paste A werden wie in Beispiel 1 unter Zusatz von 0,5 g eines Blockcopolymer-Surfactants aus Propylenoxid und Ethylenoxid mit einer mittleren Molmasse von 3800 (HLB = 1,0) (Pluronic L 101) geknetet und mit Paste B von Beispiel 1 im Gewichtsverhältnis 4 A : 1 B vermischt. Nach einigen Minuten erhält man eine gummielastische Masse. 30 Minuten nach Herstellung wird der Randwinkel zu 49° bestimmt.

Beispiel 3

100 g der Paste A werden wie in Beispiel 1 unter Zusatz von 0,5 g Polyalkylenoxid-polymethylsiloxan, Molmasse 600 (HLB = 5-8) (Silwet L-77) geknetet und mit Paste B von Beispiel 1 im Gewichtsverhältnis 4 A : 1 B vermischt. Nach einigen Minuten erhält man eine gummielastische Masse. 30 Minuten nach Herstellung wird der Randwinkel zu 34° bestimmt.

Die Wiederholung der Kontaktwinkelmessung nach 7 Monaten Lagerzeit (bei 23°C) führt zu einem Randwinkel von 33°. Dies zeigt, daß der Randwinkel unverändert geblieben ist und die erfindungsgemäße Masse eine sehr gute Lagerbeständigkeit besitzt.

Beispiel 4 (Vergleichsbeispiel)

Es werden 60,10 g eines Aziridinopolyethers, der gemäß Beispiel 12 der DE-B-17 45 810 erhalten wurde, mit 3,2 g Normulgen, 10,7 g eines Blockcopolymer-Surfactants aus Propylenoxid und Ethlyenoxid mit einer mittleren Molmasse von 6500 (HLB = 15,0) (Pluroinic P 105), 16,0 g eines Blockcopolymer-Surfactants aus Propylenoxid und Ethylenoxid mit einer mittleren Molmasse von 4400 (HLB = 0,5) (Pluronic L 121), 2,1 g Telamid, 7,5 g hydriertem Palmöl und 17,9 g Kieselgur vermischt. Auf diese Weise werden 117,5 g einer Paste C erhalten. 40 g dieser Paste C werden mit 10 g der Paste B von Beispiel 1 vollständig vermischt. Nach einigen Minuten erhält man eine gummielastische Masse. 30 Minuten nach Herstellung wird der Randwinkel zu 18° bestimmt. Die Wasseraufnahme beträgt 10,05 %, das Wasserrückhaltevermögen 7,40 %, die Dimensionsänderung + 0,94 %.

Beispiel 5

Es werden 100 g einer Paste A entsprechend Beispiel 1 hergestellt. Weiterhin werden 32,7 g eines Sulfoniumsalzes, das gemäß Beispiel 31 der DE-C-25 15 593 erhalten wurde, 22,2 g Acetyltributylcitrat, 5,8 g eines Blockcopolymer-Surfactants aus Propylenoxid und Ethylenoxid mit einer mittleren Molmasse von 6500 (HLB = 15,5) (Pluronic P 105), 10,1 g pyrogene Kieselsäure, 28,5 g Kieselgur und 0,7 g Pigmente zu insgesamt 100 g einer Paste D verknetet. Die beiden Pasten werden im Gewichtsverhältnis 7 A : 1 D miteinander vollständig vermischt. Nach einigen Minuten erhält man eine gummielastische Masse. 30 Minuten nach Herstellung wird der Randwinkel zu 51° bestimmt. Die Wasseraufnahme beträgt 1,15 %, das Wasserrückhaltevermögen 0,20 %, die Dimensionsänderung 0,00 %. Nach 10 Minuten Desinfektion eines Prüfkörpers in einem Desinfektionstauchbad (Impresept, ESPE), anschließendem Spülen unter kaltem Wasser und 2-stündiger Lagerung an Luft bei 23°C und 50 % relativer Luftfeuchtigkeit wird der Randwinkel zu 52° bestimmt.

Beispiele 6 bis 18

100 g der Paste A entsprechend Beispiel 1 werden mit der jeweils nachfolgend angegebenen Menge x an Hydrophilierungsmittel zu einer hydrophilierten Paste Ax verknetet. Auf diese Weise werden 100 + x g einer Paste Ax erhalten. Weiterhin werden 100 g einer Paste D wie in Beispiel 5 beschrieben hergestellt. Die beiden Pasten werden im Gewichtsverhältnis 7 Ax : 1 D vollständig miteinander vermischt. Nach einigen Minuten erhält man eine gummielastische Masse. 30 Minuten nach Herstellung wird der Randwinkel bestimmt. Die Meßwerte für den 10-Sekunden-Randwinkel sind in Tabelle 2 aufgeführt.

Beispiel 6

100 g A + 5,17 g Fettalkoholpolyalkylenoxidmethylether (Kokosfettverschnitt) (Rewopal MT 2540). 30 Minuten nach Herstellung wird der Randwinkel zu 28° bestimmt. Die Wasseraufnahme beträgt 1,48 %, das Wasserrückhaltevermögen 0,43 %, die Dimensionsänderung + 0,08 %.

Beispiel 7

100 g A + 1,96 g eines Blockcopolymer-Surfactants aus Propylenoxid und Ethylenoxid mit einer mittleren Molmasse von 3800 (HLB = 1,0) (Pluronic L 101). 30 Minuten nach Herstellung wird der Randwinkel zu 22° bestimmt. Die Wasseraufnahme beträgt 1,57 %, das Wasserrückhaltevermögen 0,51 %, die Dimensionsänderung + 0,14 %.

Beispiel 8

100 g A + 1,44 g eines Blockcopolymer-Surfactants aus Propylenoxid und Ethylenoxid mit einer mittleren Molmasse von 6500 (HLB = 15,0) (Pluronic P 105).

Beispiel 9

100 g A + 5,17 g eines Blockcopolymer-Surfactants aus Propylenoxid und Ethylenoxid mit einer mittleren Molmasse von 2200 (HLB = 16,0) (Pluronic L 44).

Beispiel 10

100 g A + 5,17 g eines Blockcopolymer-Surfactants aus Propylenoxid und Ethylenoxid mit einer mittleren Molmasse von 2900 (HLB = 15,0) (Pluronic L 64).

Beispiel 11

100 g A + 1 g eines Nonylphenolethoxylats mit 9 EO-Einheiten (HLB = 13,0) (Antarox CO-630).

Beispiel 12

100 g A + 1 g eines Polyalkylenoxid-polymethylsiloxans mit EO/PO-Einheiten, Molmasse 20000, methoxytherminiert (HLB = 9-12) (Silwet L-7001).

Beispiel 13

100 g A + 1 g eines Polyalkylenoxid-polymethylsiloxans mit EO-Einheiten, Molmasse 4000 (HLB = 13-17) (Silwet L-7604).

Beispiel 14

100 g A + 1 g eines Fettalkoholpolyalkylenoxidmethylethers (Rewopal MT 5722).

Beispiel 15

100 g A + 5 g eines Blockcopolymer-Surfactants aus Propylenoxid und Ethylenoxid mit einer mittleren Molmasse von 4150 (HLB = 16,5) (Pluronic P 75).

Beispiel 16

100 g A + 1 g eines fluorierten Alkylalkoxylats (Fluorad FC-430).

Beispiel 17

100 g A + 1 g eines fluorierten Alkylesters (Fluorad FC-430).

Beispiel 18

100 g A + 1 g eines Nonylphenolethoxylat mit 4 EO-Gruppen (HLB = 8,8) (Antarox CO-430).

Beispiel 19

Aus der Polyetherzusammensetzung gemäß Beispiel 5 werden Prüfkorper zur Bestimmung der Dimensionsstabilität gemäß ADA 19 hergestellt. Die Dimensionsänderung nach ADA 19 wird nach 1 Stunde zu -0,10 %, nach 24 Stunden zu -0,24 % bestimmt. Der 10-Sekunden-Randwinkel beträgt 51°. Diese Prüfkörper werden anschließend in eine frisch zubereitete kommerzielle Desinfektionslösung (CoeCide XL plus, GC America) gelegt und bei 23°C gemäß den Herstellerangaben 20 Minuten in dieser Lösung belassen. Danach werden die Prüfkörper unter fließendem kalten Wasser kurz abgespült und 2 Stunden an Luft von 23°C und 50 % relativer Luftfeuchtigkeit getrocknet. Anschließend wird dieser Vorgang noch 2 x wiederholt, so daß die Prüferkörper eine dreimalige Desinfektion erfahren haben. Nach der letzten Desinfektion werden wiederum die Dimensionsänderung und der Randwinkel bestimmt. Die Dimensionsänderung beträgt +0,05 %, der Randwinkel wird zu 52° bestimmt. Dieser Versuch zeigt, daß die erfindungsgemäße Polyetherzusammensetzung 3 x desinfiziert werden kann und der Randwinkel sowie die Dimensionsstabilität erhalten bleiben.

Beispiel 20

Aus der Polyetherzusammensetzung gemäß Beispiel 7 werden Prüfkörper zur Bestimmung der Dimensionsstabilität gemäß ADA 19 hergestellt. Die Dimensionsänderung gemäß ADA 19 wird nach einer Stunde zu -0,15 % und nach 24 Stunden zu -0,25 % bestimmt. Der 10-Sekunden-Randwinkel beträgt 22°. Diese Prüfkörper werden anschließend in eine frisch zubereitete kommerzielle Desinfektionslösung (Banicide, Pascal) gelegt und bei 23°C gemäß den Herstellerangaben 10 Minuten in dieser Lösung belassen. Danach werden die Prüfkörper unter fließendem kalten Wasser kurz abgespült und 2 Stunden an Luft von 23°C und 50 % relativer Luftfeuchtigkeit getrocknet. Anschließend wird dieser Vorgang noch 2 x wiederholt, so daß die Prüfkörper eine dreimalige Desinfektion erfahren haben. Nach der letzten Desinfektion werden wiederum die Dimensionsänderung und der Randwinkel bestimmt. Die Dimensionsänderung beträgt +0,15 %, der Randwinkel wird zu 23° bestimmt. Dieser Versuch zeigt, daß die erfindungsgemäße Polyetherzusammensetzung 3 x desinfiziert werden kann und der Randwinkel sowie die Dimensionsstabilität erhalten bleiben.

Beispiel 21

Zwei handelsübliche hydrophilierte additionsvernetzende Silikonabdruckmassen, Imprint (3M) und Reprosil (Dentsply) werden im direkten Vergleich zu den erfindungsgemäßen Zusammensetzungen der Beispiele 2, 3, 5 und 7 in einem handelsüblichen Desinfektions-Tauchbad (Impresept, ESPE) desinfiziert und der Einfluß des Desinfektionsvorgangs auf den Randwinkel der Abdruckmassen geprüft. Dazu werden von allen Abformmassen Prüferkörper hergestellt und 30 Minuten nach Anmischen deren 10-Sekunden-Randwinkel vor Desinfektion bestimmt (Tabelle 3). Anschließend werden die Prüfkörper 10 Minuten in dem handelsüblichen Desinfektions-Tauchbad belassen. Die Prüfkörper werden daraufhin unter fließendem kaltem Wasser kurz gespült und 2 Stunden an Luft von 23°C und 50 % relativer Luftfeuchtigkeit trocknen gelassen. Danach wird wiederum der 10-Sekunden-Randwinkel nach Desinfektion bestimmt. Das Ergebnis in Tabelle 3 zeigt, daß die dem Stand der Technik entsprechenden hydrophilierten Silikone nach nur einer Desinfektion bereits eine deutliche Erhöhung des Randwinkels erfahren und somit an Benetzbarkeit im anschließenden Ausgießvorgang mit Gipssuspension deutlich einbüßen. Die erfindungsgemäßen Zusammensetzungen hingegen zeigen keine oder nur eine geringfügige Veränderung des Randwinkels und besitzen eine auch nach der Desinfektion gleichbleibend gute Benetzbarkeit.

TABELLE 1

| | | Beispiel | Hydrophilierungsmittel | Gehalt (in % der Gesamtmasse) | 10-Sek.-RW | Wasser-Auf-nahme,% | Dimensionsän-derung, % |
|---|---|---|---|---|---|---|---|
| | 1 | (Vergleichsbeispiel) | - | 0 | 64° | 1,05 | 0,18 ± 0,00 |
| | 2 | (Vergleichsbeispiel) | Blockcopolymer-Surfactant | 18,18 | 18° | 10,05 | 7,40 + 0,94 |
| | 5 | (erfindungsgemäß) | Blockcopolymer-Surfactant | 0,73 | 51° | 1,15 | 0,20 ± 0,00 |
| | 6 | (erfindungsgemäß) | Fettalkoholpo-lyalkylenoxid-methylether | 5,03 | 28° | 1,48 | 0,43 + 0,08 |
| | 7 | (erfindungsgemäß) | Blockcopolymer-Surfactant | 2,41 | 22° | 1,57 | 0,51 + 0,14 |

Tabelle 1: Einfluß von Art/Gehalt an Hyrophilierungsmittel auf Randwinkel, Wasseraufnahme und -abgabe sowie die Dimensionsänderung

TABELLE 2

| | Beispiel | Hydrophilierungsmittel | Gehalt in % | 10 Sek.-RW |
|---|---|---|---|---|
| 1 | (Vergleichsbeispiel) | - | 0 | 64° |
| 2 | (erfindungsgemäß) | Blockcopolymer-Surfactant | 0,40 | 49° |
| 3 | dto | Polyalkylenoxidpolymethylsiloxan | 0,40 | 34° |
| 4 | (Vergleichsbeispiel) | Blockcopolymer-Surfactant | 18,18 | 18° |
| 5 | (erfindungsgemäß) | Blockcopolymer-Surfactant | 0,73 | 51° |
| 6 | dto | Fettalkoholpolyalkylenoxidmethylether | 5,03 | 28° |
| 7 | dto | Blockcopolymer-Surfactant | 2,41 | 22° |
| 8 | dto | Blockcopolymer-Surfactant | 1,97 | 40° |
| 9 | dto | dto | 5,03 | 30° |
| 10 | dto | dto | 5,03 | 25° |
| 11 | dto | Nonylphenolethoxylat | 1,59 | 26° |
| 12 | dto | Polyalkylenoxidpolymethylsiloxan | 1,59 | 35° |
| 13 | dto | dto | 1,59 | 44° |

TABELLE 2 (fortgesetzt)

|  | Beispiel | Hydrophilierungsmittel | Gehalt in % | 10 Sek.-RW |
|---|---|---|---|---|
| 14 | dto | Fettalkoholpolyalkylenoxidmethylether | 1,59 | 25° |
| 15 | dto | Blockcopolymer-Surfactant | 4,89 | 30° |
| 16 | dto | Fluoriertes Alkylalkoxylat | 1,59 | 42° |
| 17 | dto | Fluorierter Alkylester | 1,59 | 42° |
| 18 | dto | Nonylphenolethoxylat | 1,59 | 53° |
| Tabelle 2: Einfluß von Art/Gehalt an Hydrophilierungsmittel auf den Randwinkel | | | | |

TABELLE 3

| Abformmasse | Zusammensetzung | 10-Sek.-RW vor Desinfektion | 10-Sek.-RW nach Desinfektion | % RW-Erhöhung |
|---|---|---|---|---|
| Imprint, 3M | USP 86022442 WO 87/03001 | 55° | 75° | 36 |
| Reprosil, Dentsply | EP-B1-0 231 420 | 45° | 65° | 44 |
| Beispiel 2 |  | 49° | 49° | 0 |
| Beispiel 3 |  | 34° | 36° | 6 |
| Beispiel 5 |  | 51° | 52° | 2 |
| Beispiel 7 |  | 22° | 22° | 0 |
| Tabelle 3: Verhalten von hydrophilierten Silikon- und Polyetherabformmassen bei der Desinfektion | | | | |

**Patentansprüche**

1. Gummielastische Masse auf der Basis vulkanisierbarer Polyether mit Aziridinogruppen, dadurch gekennzeichnet, daß sie in fertig vulkanisierter und auspolymerisierter Form 0,1 bis 15 Gew.-%, bezogen auf das Gesamtgewicht der Masse, wenigstens eines eine hydrophile Natur verleihenden Mittels aus der Gruppe bestehend aus hydrophilen Silikonölen, fluorierten Kohlenwasserstoffen, Blockcopolymeren von Ethylenoxid/Propylenoxid, Fettalkoholderivaten, Alkylphenolderivaten, Fettaminen, Aminoxiden, Fettsäureglykol- und -glycerinderivaten, Fettsäuren und Fettsäuremonoestern enthält.

2. Masse nach Anspruch 1, dadurch gekennzeichnet, daß sie 0,2 bis 10, vorzugsweise 0,5 bis 5 Gew.-% des eine hydrophile Natur verleihenden Mittels enthält.

3. Masse nach den Ansprüchen 1 oder 2, dadurch gekennzeichnet, daß sie in fertig vulkanisierter und auspolymerisierter Form einen 10-Sekunden-Randwinkel von weniger als 55°, vorzugsweise weniger als 45° und insbesondere weniger als 35° aufweist.

4. Masse nach den Ansprüchen 1 bis 3, dadurch gekennzeichnet, daß sie in fertig vulkanisierter und auspolymerisierter Form nach zweimaliger 10- bis 20-minütiger Desinfektion in wäßriger Desinfektionslösung einen 10-Sekunden-Randwinkel von weniger als 55°, vorzugsweise weniger als 45° und insbesondere weniger als 35° aufweist.

5. Masse nach den Ansprüchen 1 bis 4, dadurch gekennzeichnet, daß die hydrophilen Silikonöle Silikonpolyethermoleküle mit mindestens einem hydrophilen Polyetheranteil sind.

6. Masse nach den Ansprüchen 1 bis 4, dadurch gekennzeichnet, daß sie als eine hydrophile Natur verleihendes Mittel einen Fettalkohol enthält, der mit Ethylen- und/oder Propylenoxid umgesetzt und gegebenenfalls methyliert oder mit einer Monocarbonsäure verestert ist.

**7.** Masse nach den Ansprüche 1 bis 4, dadurch gekennzeichnet, daß sie als eine hydrophile Natur verleihendes Mittel ein Blockcopolymeres des Propylenoxids und Ethylenoxids enthält.

**8.** Polyetherabformmasse gemäß Ansprüchen 1 bis 7, zur Verwendung für die Zahn-, Schleimhaut- und Modellabformung.

**9.** Verfahren zur Herstellung von Polyetherabformmassen gemäß einem der Ansprüche 1-8, enthaltend

a) Polyether mit Aziridinogruppen und eine SiH-Komponente,
b) für Polyether übliche Polymerisationskatalysatoren,
c) wenigstens ein eine hydrophile Natur verleihendes Mittel aus der Gruppe bestehend aus hydrophilen Silikonölen, fluorierten Kohlenwasserstoffen, Blockcopolymeren von Ethylenoxid/Propylenoxid, Fettalkoholderivaten, Alkylphenolderivaten, Fettaminen, Aminoxiden, Fettsäureglykol- und -glycerinderivaten, Fettsäuren und Fettsäuremonoestern,
d) gegebenenfalls übliche Aktivatoren, Beschleuniger, Verzögerer und Stabilisatoren,
e) gegebenenfalls übliche Lösungsmittel oder Weichmacher,
f) gegebenenfalls weitere übliche pyrogene oder gefällte Kieselsäuren, Füllstoffe, Desinfektionsmittel und weitere übliche Zusatzstoffe,

dadurch gekennzeichnet, daß durch Vermischen von gegebenenfalls Teilmengen von Bestandteilen a) bis f) mit Ausnahme von b) eine Basispaste A, durch Vermischen von gegebenenfalls Teilmengen von Bestandteilen a) bis f), mit Ausnahme von a), eine Katalysatorpaste B hergestellt wird, und zur Herstellung der gebrauchsfertigen Abformmasse äquivalente Mengen A und B miteinander vermischt werden.

**Claims**

**1.** Rubber-elastic composition based on vulcanizable, polyethers with aziridino groups, characterized in that, in the completely vulcanized and completely polymerized form, it contains 0.1 to 15 wt.-%, relative to the total weight of the composition, of at least one agent imparting a hydrophilic nature from the group consisting of hydrophilized silicone oils, fluorinated hydrocarbons, block copolymers of ethylene oxide / propylene oxide, fatty alcohol derivatives, alkyl phenol derivatives, fatty amines, amine oxides, fatty acid glycol and glycerin derivatives, fatty acids and fatty acid monoesters.

**2.** Composition according to claim 1, characterized in that it contains 0.2 to 10, preferably 0.5 to 5 wt.-% of the agent imparting a hydrophilic nature.

**3.** Composition according to claims 1 or 2, characterized in that, in the completely vulcanized and completely polymerized form, it has a 10-sec wetting angle of less than 55°, preferably less than 45° and especially less than 35°.

**4.** Composition according to claims 1 to 3, characterized in that, in the completely vulcanized and completely polymerized form, after two 10- to 20-minute disinfections in aqueous disinfection solution, it has a 10-sec wetting angle of less than 55°, preferably less than 45° and especially less than 35°.

**5.** Composition according to claims 1 to 4, characterized in that the hydrophilic silicone oils are silicone polyether molecules with at least one hydrophilic polyether portion.

**6.** Composition according to claims 1 to 4, characterized in that it contains, as agent imparting a hydrophilic nature, a fatty alcohol which is reacted with ethylene and/or propylene oxide and optionally methylated or esterified with a monocarboxylic acid.

**7.** Composition according to claims 1 to 4, characterized in that it contains as an agent imparting a hydrophilic nature a block copolymer of propylene oxide and ethylene oxide.

**8.** Polyether modelling composition according to claims 1 to 8 for use for teeth, mucous membrane and model impressions.

**9.** Process for the production of polyether modelling compositions according to claims 1-8, containing

a) polyether with aziridino groups and an SiH component,

b) polymerization catalysts usual for polyethers,

c) at least one agent imparting a hydrophilic nature from the group consisting of hydrophilic silicone oils, fluorinated hydrocarbons, block copolymers of ethylene oxide / propylene oxide, fatty alcohol derivatives, alkyl phenol derivatives, fatty amines, amine oxides, fatty acid glycol and glycerin derivatives, fatty acids and fatty acid monoesters,

d) optionally usual activators, accelerators, retarders and stabilizers,

e) optionally usual solvents or plasticizers,

f) optionally other usual pyrogenic or precipitated silicic acids, fillers, disinfectants and other usual additives,

characterized in that a base paste A is produced by mixing optionally partial quantities of constituents a) to f) except b), a catalyst paste B is produced by mixing optionally partial quantities of constituents a) to f) except a), and for producing the ready-to-use modelling composition equivalent quantities of A and B are mixed together.

## Revendications

1. Mélange de gomme élastique à base de polyéthers vulcanisables avec des groupes aziridino, caractérisé en ce que, sous forme complètement vulcanisée et polymérisée, il contient de 0,1 à 15% en poids, ramené au poids total du mélange, d'au moins un agent conférant une nature hydrophile choisi dans le groupe comprenant des huiles de silicone hydrophiles, des hydrocarbures fluorés, des copolymères en masse d'oxyde d'éthylène et oxyde de propylène, des dérivés d'alcools gras, des dérivés d'alkylphénols, des amines grasses, des oxydes d'amines, des dérivés glycoliques et glycéroliques d'acides gras, des acides gras et des monoesters d'acides gras.

2. Mélange selon la revendication 1, caractérisé en ce qu'il contient de 0,2 à 10, de préférence 0,5 à 5% en poids de l'agent conférant une nature hydrophile.

3. Mélange selon la revendication 1 ou la revendication 2, caractérisé en ce que, sous forme complètement vulcanisée et polymérisée, il présente un angle de mouillage à 10 secondes de moins de 55°, de préférence moins de 45° et en particulier moins de 35°.

4. Mélange selon les revendications 1 à 3, caractérisé en ce que, sous forme complètement vulcanisée et polymérisée, après désinfection à deux reprises pendant 10 à 20 minutes dans une solution désinfectante aqueuse, il présente un angle de mouillage à 10 secondes de moins de 55°, de préférence moins de 45° et en particulier moins de 35°.

5. Mélange selon les revendications 1 à 4, caractérisé en ce que les huiles de silicone hydrophiles sont des molécules de polyéthers de silicone avec au moins une fraction de polyéthers hydrophiles.

6. Mélange selon les revendications 1 à 4, caractérisé en ce que, comme agent conférant une propriété hydrophile, il contient un alcool gras qui a été mis à réagir avec de l'oxyde d'éthylène et/ou de l'oxyde de propylène et le cas échéant méthylé, ou bien estérifié avec un acide monocarboxylique.

7. Mélange selon les revendications 1 à 4, caractérisé en ce que, comme agent conférant une propriété hydrophile, il contient un copolymère en masse d'oxyde de propylène et d'oxyde d'éthylène.

8. Mélange de polyéthers pour prise d'empreintes selon les revendications 1 à 7, pour utilisation dans la prise d'empreintes de dents, de muqueuses et de modèles.

9. Procédé de fabrication de mélanges de polyéthers pour prise d'empreintes selon l'une des revendications 1 à 8, contenant :

a) des polyéthers avec des groupes aziridino et un composant SiH,

b) des catalyseurs de polymérisation usuels pour les polyéthers,

c) au moins un agent conférant une nature hydrophile choisie dans le groupe comprenant des huiles de silicone hydrophiles, des hydrocarbures fluorés, des copolymères en masse d'oxyde d'éthylène/oxyde de propylène, des dérivés d'alcools gras, des dérivés d'alkylphénols, des amines grasses, des oxydes d'amines, des dérivés glycoliques et glycéroliques d'acides gras, des acides gras et des monoesters d'acides gras,

d) le cas échéant des activateurs, accélérateurs, retardateurs et stabilisateurs usuels,

e) le cas échéant des solvants ou plastifiants usuels,

f) le cas échéant d'autres acides siliques usuels pyrogénés ou précipités, des matières de charge, des agents désinfectants, et d'autres additifs usuels,

caractérisé en ce que l'on prépare une pâte de base A par mélangeage de quantités le cas échéant partielles des composants a) à f) à l'exception de b), on prépare une pâte de catalyseur B par mélangeage de quantités le cas échéant partielles des composants a) à f) à l'exception de a), et on mélange entre elles des quantités équivalentes de A et de B afin de préparer le mélange de prise d'empreintes prêt à l'emploi.